(19) Europäisches Patentamt
European Patent Office
Office européen des brevets

(11) EP 3 858 353 B1

(12) **EUROPEAN PATENT SPECIFICATION**

(45) Date of publication and mention of the grant of the patent:
**07.02.2024 Bulletin 2024/06**

(21) Application number: **18934879.0**

(22) Date of filing: **05.11.2018**

(51) International Patent Classification (IPC):
***A61K 9/08*** (2006.01) ***A61K 31/46*** (2006.01)
***A61K 9/00*** (2006.01) ***A61K 47/02*** (2006.01)
***A61K 47/04*** (2006.01) ***A61K 47/12*** (2006.01)
***A61K 47/38*** (2006.01) ***A61P 27/10*** (2006.01)

(52) Cooperative Patent Classification (CPC):
**A61P 27/10; A61K 9/0048; A61K 9/08; A61K 31/46; A61K 47/02; A61K 47/12**

(86) International application number:
**PCT/CN2018/113969**

(87) International publication number:
**WO 2020/062445 (02.04.2020 Gazette 2020/14)**

(54) **PHARMACEUTICAL COMPOSITION FOR PREVENTING AND TREATING NITM AND PHARMACEUTICAL USE THEREOF**

PHARMAZEUTISCHE ZUSAMMENSETZUNG ZUR VORBEUGUNG UND BEHANDLUNG VON NITM UND IHRE PHARMAZEUTISCHE VERWENDUNG

COMPOSITION PHARMACEUTIQUE POUR PRÉVENIR ET TRAITER LA NITM ET UTILISATION PHARMACEUTIQUE ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2018 CN 201811119899**

(43) Date of publication of application:
**04.08.2021 Bulletin 2021/31**

(73) Proprietor: **Shenyang Xingqi Pharmaceutical Co., Ltd.**
**Shenyang, Liaoning 110167 (CN)**

(72) Inventors:
- **WANG, Ningli**
  **Shenyang, Liaoning 110167 (CN)**
- **LIU, Jidong**
  **Shenyang, Liaoning 110167 (CN)**
- **GUO, Lei**
  **Shenyang, Liaoning 110167 (CN)**
- **YANG, Qiang**
  **Shenyang, Liaoning 110167 (CN)**
- **GAO, Kun**
  **Shenyang, Liaoning 110167 (CN)**
- **LI, Linwei**
  **Shenyang, Liaoning 110167 (CN)**
- **GU, Hong**
  **Shenyang, Liaoning 110167 (CN)**
- **SUN, Yang**
  **Shenyang, Liaoning 110167 (CN)**
- **FU, Le**
  **Shenyang, Liaoning 110167 (CN)**

(74) Representative: **Colombo, Stefano Paolo et al**
**Marchi & Partners S.r.l.**
**Via Vittor Pisani, 13**
**20124 Milano (IT)**

(56) References cited:
**CN-A- 108 338 969** **US-A1- 2007 254 914**
**US-A1- 2015 366 854**

- **WU PEI-CHANG ET AL: "Update in myopia and treatment strategy of atropine use in myopia control", EYE, NATURE PUBLISHING GROUP, GB, vol. 33, no. 1, 11 June 2018 (2018-06-11), pages 3-13, XP037027706, ISSN: 0950-222X, DOI: 10.1038/S41433-018-0139-7 [retrieved on 2018-06-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 858 353 B1

## Description

### Technical Field

The invention belongs to the field of medicine and relates to a pharmaceutical composition for preventing and treating NITM and a medical use thereof. Specifically, the pharmaceutical composition is an ophthalmic pharmaceutical composition such as an ophthalmic preparation.

### Background Art

Myopia means that parallel light rays from a distance are focused in front of the photoreceptor layer of retina when the adjustment function is not used. In short, parallel light rays are focused in front of the retina when the eye is at rest. There is no uniform international standard that how much away from 0D the refraction is to be considered myopia. It is more practical to take -0.25D or 0.50D as the standard (see "Myopia", edited by Hu Daning, et al, People's Medical Publishing House, First edition, June 2009, page 3).

The Ophthalmology Refractive Optics Group, Ophthalmology Branch of Chinese Medical Association formulated the "Definition and Classification Criteria for True and False Myopia" in 1985, which divided myopia into three categories: false myopia, true myopia and half-true myopia (see "Myopia", edited by Hu Daning, et al, People's Medical Publishing House, First edition, June 2009, pages 25-26). In addition, myopia may also be divided into false myopia, true myopia and pathological myopia (see " Blue Book 2015 for Myopia Prevention and Control and Blindness Prevention Model", edited by Li Jianjun, People's Medical Publishing House, First edition, February 2016, pages 8-9), or myopia may be divided into pseudo-myopia, true myopia and mixed myopia (see "Ophthalmology Refractive Optics", edited by Xu Guangdi, Military Medical Science Press, First edition, June 2005, pages 71 -72; "Ophthalmology and Optometry", edited by Wang Yuliang and Li Kai, People's Military Medical Press, First edition, August 2008, pages 399-401, "Classification of myopia").

Pseudo-myopia, also known as accommodative myopia or functional myopia, refers to a kind of myopia under normal conditions, which may disappear after using an accommodating paralysis drug (1% atropine eye drops, three times a day for three consecutive days; or once a day for seven consecutive days) and appear as emmetropia or hyperopia; after the effect of the paralysis drug disappears, myopia appears again quickly. In China, pseudo-myopia accounts for about 5% to 10% of adolescent myopia, and appears mainly as myopia with a course of less than one year and a diopter below -1.00D. It is generally believed that pseudo-myopia is the primary stage of the occurrence and development of myopia (see "Myopia", edited by Hu Daning, et al, People's Medical Publishing House, June 2009, First edition, page 28; "Definition and Classification Criteria for True and False Myopia", formulated by the Ophthalmology Refractive Optics Group, Ophthalmology Branch of Chinese Medical Association).

Near-work Induced Transient Myopia (NITM) is a kind of minor and temporary shifting of far point to the front of eye due to the lens's inability to quickly and effectively reduce its own refractive power after a period of near-work, it is known to involve the accommodative lag phenomenon associated with the interaction between sympathetic nerve and parasympathetic nervous systems. NITM refers to a change in diopter (spherical equivalent degree: spherical diopter + 1/2 cylindrical diopter) after near-work, and its value is the difference between the refractive diopter after near-work and the refractive diopter before near-work (see Lin Zhong et al. Research status of transient myopia induced by near-work, Chinese Journal of Ophthalmology, July 2012, Volume 48, Issue 7, page 657). Patent document US 2015/0366854 discloses ophthalmic compositions comprising atropine and borates.

Regarding NITM, it has not drawn enough attention yet, and there is no corresponding effective treatment or prevention method.

### Contents of the Invention

After in-depth research and creative work, the inventors surprisingly found that low-concentration atropine can effectively treat NITM, and the therapeutic effect shows a dose-dependent pharmacodynamic relationship (0.001% to 0.2%). The inventors further found that the therapeutic effect is better in borate system than phosphate buffer system, and better in osmosis adjustment using polyols than using inorganic salt such as sodium chloride, and the best effect is achieved especially when only boric acid is used to adjust pH value and osmotic pressure. The pharmaceutical composition or ophthalmic preparation of the present invention may also comprise a thickener used in conventional ophthalmic preparations, preferably hydroxypropyl cellulose, and the pharmaceutical composition or ophthalmic preparation may or may not comprise a bacteriostatic agent.

Therefore, the following invention is provided:

One aspect of the present invention relates to a pharmaceutical composition, comprising atropine or a pharmaceutically acceptable salt thereof in an amount of 0.001% weight to 0.2% weight, and one or more pharmaceutically acceptable

excipients; wherein, the pharmaceutical composition has a pH value of 4.5 to 5.5; the pharmaceutical composition comprises a pH adjusting agent in an amount of 0.05% weight to 5% weight, and the pH adjusting agent is one or more selected from a group consisting of boric acid and borate.

The pH adjusting agent may be used or added in form of a solid, or used or added in form of a buffer/buffer system (e.g., borate buffer, etc.)

Regarding the pH adjusting agent, it is not excluded that boric acid and/or borate may also have another effect or function.

In one or more embodiments of the present invention, the pharmaceutical composition is an ophthalmic preparation, such as an ophthalmic liquid preparation (e.g., eye drop, eye wash or intraocular injection solution), ophthalmic semi-solid preparation (e.g., eye ointment, ophthalmic cream or ophthalmic gel), or ophthalmic solid preparation (e.g., eye film, eye pill or intraocular insert); optionally, the ophthalmic liquid preparation may be packaged in solid form, in which a solvent is provided separately, and a solution or suspension is prepared before use.

In one or more embodiments of the present invention, the pharmaceutical composition, wherein the atropine or a pharmaceutically acceptable salt thereof has a concentration or content of 0.001% to 0.1%, preferably 0.005% to 0.05%, more preferably 0.005% to 0.02%, more preferably 0.005% to 0.015% or 0.008% to 0.012%, such as 0.008%, 0.009%, 0.010%, 0.011% or 0.012%, particularly preferably 0.01%; preferably, the pharmaceutically acceptable salt of atropine is atropine sulfate.

In one or more embodiments of the present invention, the pharmaceutical composition, wherein the pharmaceutical composition has a pH of preferably4.5 to 5.5, such as 4.5 to 5.0, 5.5 to 5.5, 4.5, 4.6, 4.7, 4.8, 4.9, 5.0, 5.1, 5.2, 5.3, 5.4 or 5.5.

In one or more embodiments of the present invention, the pharmaceutical composition, wherein:

the pH adjusting agent has a content of 0.5% to 5%, more preferably 1% to 3%, further preferably 1.5% to 2.5%, 1.5% to 2.0%, 1.5% to 2.25% or 1.75% to 2.25%, such as 1.5%, 1.6%, 1.7%, 1.75%, 1.8%, 1.9%, 2.0%, 2.1%, 2.2%, 2.25%, 2.3%, 2.4% or 2.5%, particularly preferably 1.75% to 2.0%;
preferably, the borate salt is sodium tetraborate

In one or more embodiments of the present invention, the pharmaceutical composition, wherein the pharmaceutical composition comprises boric acid in an amount of 0.5% to 3%, 1% to 3%, 1.5% to 2.5%, 1.5% to 2.25% , 1.5% to 2.0%, 1.75% to 2.25% or 1.75% to 2%, such as 1.5%, 1.6%, 1.7%, 1.75%, 1.8%, 1.9%, 2.0%, 2.1%, 2.2%, 2.25 %, 2.3%, 2.4% or 2.5%.

In one or more embodiments of the present invention, the pharmaceutical composition, wherein the pharmaceutical composition further comprises any one or more items selected from the following items 1) to 5) (e.g., any 2 items, 3 items, 4 items or 5 items among them):

1) a thickener, and the thickener is any one or more selected from a group consisting of hypromellose, sodium carboxymethyl cellulose and sodium hyaluronate; preferably, the thickener has a content of 0.01 % to 5%, preferably 0.5% to 3%, more preferably 0.5% to 1.5%, such as 0.5%, 0.6%, 0.7%, 0.8%, 0.9%, 1.0%, 1.1%, 1.2%, 1.3%, 1.4 % or 1.5%; particularly preferably 0.8% to 1.2%.
2) an osmotic pressure regulator, for example, any one or more selected from a group consisting of glycerin, mannitol, propylene glycol, sodium chloride and potassium chloride;
3) a preservative, for example, any one or more selected from a group consisting of benzalkonium chloride, parabens bacteriostatic agents, and polyquaterniums;
4) a stabilizer, for example, any one or more selected from a group consisting of disodium edetate and calcium sodium edetate;
5) an appropriate amount of water.

In one or more embodiments of the present invention, the pharmaceutical composition comprises no preservative, and/or no stabilizer.

In one or more embodiments of the present invention, the pharmaceutical composition, wherein besides the active ingredient (atropine and/or pharmaceutically acceptable salt thereof, such as atropine sulfate) and excipients, the rest is water.

In one or more embodiments of the present invention, the pharmaceutical composition, wherein the ingredients and content thereof in the pharmaceutical composition are selected from any one of the following groups (1) to (6):

(1)

| | |
|---|---|
| Atropine sulfate | 0.01 parts by weight |

(continued)

| | |
|---|---|
| Hypromellose | 0.5 to 1.2 parts by weight |
| Sodium hyaluronate | 0 to 0.05 parts by weight |
| Boric acid | 1.5 to 2.0 parts by weight |
| Disodium edetate | 0.005 to 0.1 parts by weight |
| Benzalkonium chloride | 0 to 0.01 parts by weight |

Hydrochloric acid or sodium hydroxide, added to adjust pH to 4.5 to 5.5 Water for injection, added to reach 100 parts by weight;
(2)

| | |
|---|---|
| Atropine sulfate | 0.01 parts by weight |
| Hypromellose | 0.8 to 1.0 parts by weight |
| Sodium hyaluronate | 0.02 to 0.05 parts by weight |
| Boric acid | 1.75 to 2.0 parts by weight |
| Disodium edetate | 0.01 to 0.05 parts by weight |
| Benzalkonium chloride | 0 to 0.01 parts by weight |

Hydrochloric acid or sodium hydroxide, added to adjust pH to 4.5 to 5.5 Water for injection, added to reach 100 parts by weight;
(3)

| | |
|---|---|
| Atropine sulfate | 0.010 parts by weight |
| Hypromellose | 0.800 parts by weight |
| Sodium hyaluronate | 0.02 parts by weight |
| Boric acid | 2.00 parts by weight |
| Disodium edetate | 0.005 parts by weight |
| Benzalkonium chloride | 0 parts by weight |

Hydrochloric acid or sodium hydroxide, added to adjust pH to 4.5 Water for injection, added to reach 100 parts by weight;
(4)

| | |
|---|---|
| Atropine sulfate | 0.010 parts by weight |
| Hypromellose | 0.500 parts by weight |
| Sodium hyaluronate | 0.05 parts by weight |
| Boric acid | 2.00 parts by weight |
| Disodium edetate | 0.010 parts by weight |
| Benzalkonium chloride | 0.010 parts by weight |

Hydrochloric acid or sodium hydroxide, added to adjust pH to 5.0 Water for injection, added to reach 100 parts by weight;
(5)

| | |
|---|---|
| Atropine sulfate | 0.010 parts by weight |
| Hypromellose | 1.000 parts by weight |
| Sodium hyaluronate | 0 parts by weight |
| Boric acid | 1.750 parts by weight |
| Disodium edetate | 0.050 parts by weight |
| Benzalkonium chloride | 0 parts by weight |

Hydrochloric acid or sodium hydroxide, added to adjust pH to 5.5 Water for injection, added to reach 100 parts by weight;
(6)

| | |
|---|---|
| Atropine sulfate | 0.010 parts by weight |
| Hypromellose | 1.200 parts by weight |
| Sodium hyaluronate | 0 parts by weight |
| Boric acid | 2.0 parts by weight |
| Disodium edetate | 0.010 parts by weight |
| Benzalkonium chloride | 0.010 parts by weight |

Hydrochloric acid or sodium hydroxide, added to adjust pH to 5.0 Water for injection, added to reach 100 parts by weight;

The pharmaceutical composition of the present invention can be prepared by technical means known to those skilled in the art.

Another aspect of the present invention relates to a composition according to the claims, for use in the treatment and/or prevention of NITM (near-work induced transient myopia);

preferably, the pharmaceutically acceptable salt is atropine sulfate;
preferably, the pharmaceutical composition further comprises one or more pharmaceutically acceptable excipients;
preferably, the pharmaceutical composition is the pharmaceutical composition of any one of the items of the present invention.

In one or more embodiments of the present invention, the pharmaceutical composition is an ophthalmic preparation, preferably an eye drop.

In one or more embodiments of the present invention, the atropine or a pharmaceutically acceptable salt thereof, or the pharmaceutical composition containing atropine or a pharmaceutically acceptable salt thereof, wherein the eye drop is administered once a day or every other day, 1 to 2 drops each time; preferably, the eye drop is dripped into a conjunctival sac.

In one or more embodiments of the present invention, the atropine or a pharmaceutically acceptable salt thereof, or the pharmaceutical composition containing atropine or a pharmaceutically acceptable salt thereof, wherein the eye drop is administered to a subject who is a person using eyes in near distance or a person susceptible to myopia;

preferably, the person using eyes in near distance is a myopia patient or a non-myopia patient;
preferably, the person susceptible to myopia is a person aged 6 to 18.

The actual dosage and period of administration can be determined according to the subject's own physical conditions or in accordance with the doctor's advice. Under normal circumstances, it can be administered in a short or long term, such as 1 month, 2 months, 3 months, 4 months, 5 months, 6 months, 1 year, 1.5 years, 2 years, 2.5 years, 3 years or more than 3 years.

Some terms involved in the present invention are explained as follows:

The structural formula of atropine is shown in the following formula I:

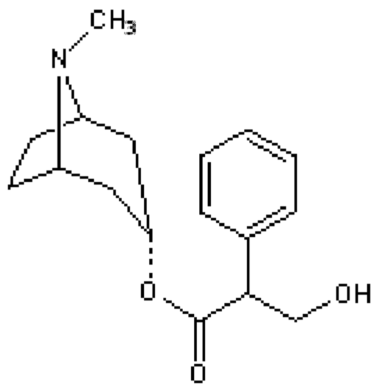

Formula I

The structure of atropine sulfate is shown in the following formula II:

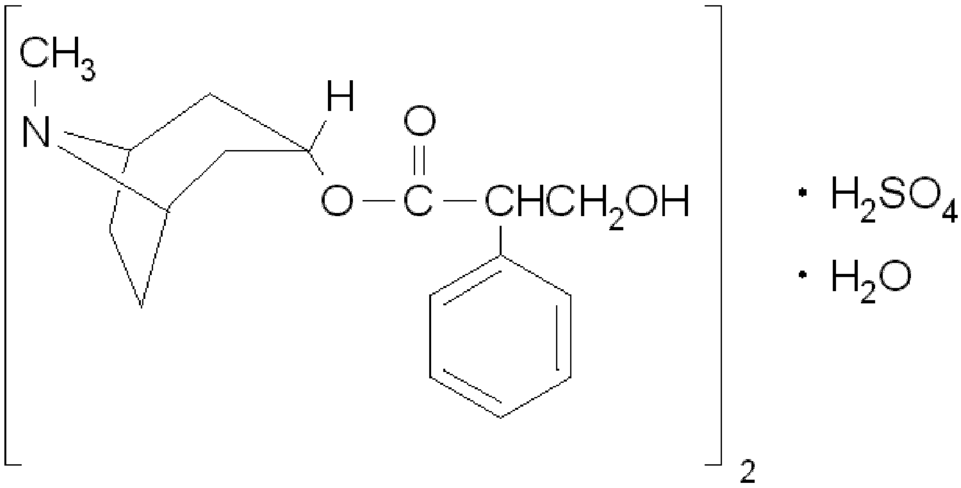

Formula II

In the present invention, the term "person using eyes in near distance" refers to a person whose eyes are relatively close to a gaze target, and who maintains continuous gaze for a period of time. In a narrow sense: for the term "person using eyes in near distance", "near distance" or "shorter distance" means that the straight line distance between the eyes and the gaze target is 10cm to 50 cm; "a period of time" refers to at least 30 minutes, at least 45 minutes, at least 1 hour, at least 1.5 hours, at least 2 hours, at least 2.5 hours, at least 3 hours, at least 4 hours, or more than 4 hours, wherein "continuous gaze" refers to uninterrupted gaze; in some individual cases, one, two or three interruptions may be allowed, but each interruption time does not exceed 5 seconds, preferably not more than 3 seconds or not more than 2 seconds. Generally, the person using eyes in near distance includes but is not limited to elementary and middle school students, office white-collar workers, persons engaged in electronic games or videos, etc.; the gaze target includes, but is not limited to, electronic display devices (mobile phone displays, computer displays, electronic readers, etc.), books, examination papers, etc. The person using eyes in near distance includes myopia patients and non-myopia patients.

In the present invention, the term "person susceptible to myopia" includes persons who are susceptible to myopia but have not yet suffered from myopia, or persons who are already suffering from myopia and are likely to further aggravate the degree of myopia. Generally speaking, persons aged 6 to 18 (children, adolescents) are susceptible to myopia because their eyes and optic nervous system are not yet fully developed, or because they are under pressure to study, or are exposed to video games or videos frequently. The person susceptible to myopia includes myopia patients and non-myopia patients, preferably myopia patients or non-myopia patients aged 6 to 18.

In the present invention, the concentration of the atropine or a pharmaceutically acceptable salt thereof (e.g., atropine sulfate), if not otherwise specified, refers to mass/volume percentage concentration. In the present invention, the content of the atropine or a pharmaceutically acceptable salt thereof (e.g., atropine sulfate), if not specifically specified, refers to mass/volume (g/ml) percentage content.

In the present invention, the content of a certain excipient material (e.g., boric acid), if not otherwise specified, refers to mass/volume (g/ml) percentage content.

The mass/volume percentage refers to the number of grams of solute contained in a volume of one hundred milliliters. The relative density of the liquid preparation involved in the present invention is about 1.0. Therefore, in the actual preparation process, it can be calculated as hundred grams per hundred milliliters. Therefore, the mass/volume (g/ml) percentage can also be approximately mass/mass (g/g) percentage.

The beneficial effects of the present invention

The present invention has achieved one or more of the following technical effects:

(1) for the pharmaceutical composition of the present invention, the use of boric acid and/or borate to adjust pH and/or osmotic pressure achieves very good effect, which is better than that of other pH adjusting agents and/or osmotic pressure adjusting agents;

(2) the use of boric acid and/or borate can enhance the effect of treating and/or preventing NITM;

(3) the use of boric acid and/or borate can reduce the mydriatic side effects of atropine to a certain extent;

(4) atropine or a pharmaceutically acceptable salt thereof (e.g., atropine sulfate), or a pharmaceutical composition containing atropine or a pharmaceutically acceptable salt thereof (e.g., atropine sulfate), can effectively treat and/or prevent NITM.

## Specific Models for Carrying Out the Invention

The embodiments of the present invention will be described in detail below in conjunction with examples, but those skilled in the art will understand that the following examples are only used to illustrate the present invention and should not be regarded as limiting the scope of the present invention. If a specific condition is not indicated in the examples, it shall be carried out in accordance with conventional condition or condition recommended by the manufacturer. The reagents or instruments used without giving their manufacturers are all conventional products that are commercially available.

Example 1: Experiment of NITM treatment (not according to the claims)

1. Experimental drug

0.01% Atropine sulfate eye drop was prepared with 0.9% saline and adjusted the pH to 6.0 with hydrochloric acid. It was used for administration in the test group.

2. Experimental method

30 Myopia patients aged 6 to 18 were selected. The selected patients who had eye diopter of -0.50DS to -5.00DS, astigmatism ≤-1.0DC; initial NITM value ≥-0.25D; normal intraocular pressure not exceeding 21mmHg, were randomly divided into 2 groups, 15 patients in each group. The test group was dripped with the aforementioned experimental drug, and the control group was dripped with 0.9% normal saline; each group was administered once a day, 1 drop per eye each time, and the aforementioned experimental drug or 0.9% normal saline was dripped into conjunctival sac before sleep for consecutive 6 months. NITM detection was performed at scheduled time points, and the initial NITM value, NITM attenuation time, and the spherical equivalent degree of glasses worn to achieve the best corrected visual acuity were recorded. The effectiveness was determined when the initial NITM value decreased by more than or equal to 0.20D, which could be used to calculate the effective rate of drug.

The drug was determined as effective in a case when the initial NITM value after administration decreased by more than or equal to 0.20D in comparison with the initial NITM at baseline.

$$\text{Effective rate} = \text{number of effective cases/ number of treated patients} \times 100\%.$$

Wherein, NITM at baseline referred to a NITM value before administration, that was, the NITM value on the $0^{th}$ day, and initial NITM at baseline was the difference between the average diopter within 10s after near-work and the diopter before near-work on the $0^{th}$ day before administration. The measurement method of NITM at baseline was consistent with the following NITM measurement method at each time point after administration.

NITM measurement: optometry was carried out by using a phoropter (non-cycloplegia), according to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again, and the initial NITM was recorded. The initial NITM was the difference between the diopter within 10s after near-work and the diopter before near-work (Table 1), the NITM attenuation time referred to the time taken for the initial NITM to decrease to zero (the attenuation time reflected the speed at which the eyes returned to their daily level after transient myopia caused by near-work. Unit: seconds).

3. Experimental results

The results were shown in Table 1 to Table 4 below.

Table 1: Effect of 0.01% atropine sulfate eye drop on initial NITM value of eyes at different time points ($\bar{x} \pm S$, n=15)

| Group | Observation time (days) | | | | |
|---|---|---|---|---|---|
| | 0 | 30 | 60 | 90 | 180 |
| Control group | 0.38±0.09 | 0.37±0.09 | 0.37±0.07 | 0.40±0.07 | 0.35±0.06 |
| Test group | 0.38±0.09 | 0.30±0.05 | 0.23±0.06 | 0.12±0.06 | 0.14±0.03 |

Table 2: Effect of 0.01% atropine sulfate eye drop on NITM attenuation time of eyes at different time points ($\bar{x} \pm S$, n=15)

| Group | Observation time (days) | | | | |
|---|---|---|---|---|---|
| | 0 | 30 | 60 | 90 | 180 |
| Control group | 140.8±12.6 | 140.9±10.8 | 143.9±11.6 | 139.8±11.4 | 142.8±11.6 |
| Test group | 138.3±13.0 | 109.7±8.4 | 92.9±8.5 | 72.5:1:11.7 | 50.5±10.7 |

Table 3: Effective rate of 0.01% atropine sulfate eye drop on NITM of eyes at different time points ($\bar{x} \pm S$, n=15)

| Group | Observation time (days) | | | | |
|---|---|---|---|---|---|
| | 0 | 30 | 60 | 90 | 180 |
| Control group | 0 | 6.67% | 0 | 0 | 0 |
| Test group | 0 | 20.00% | 33.33% | 86.67% | 80.00% |

Table 4: Effect of 0.01% atropine sulfate eye drop on spherical equivalent degree of eyes at different time points ($\bar{x} \pm S$, n=15)

| Group | Observation time (days) | | | | |
|---|---|---|---|---|---|
| | 0 | 30 | 60 | 90 | 180 |
| Control group | -2.87±1.23 | -2.86±1.23 | -2.92±1.28 | -2.97±1.22 | -3.23±1.14 |
| Test group | -2.85±1.25 | -2.85±1.25 | -2.85±1.25 | -2.9±1.25 | -2.92±1.26 |
| Note: Diopter is classified into spherical diopter (myopia, hyperopia; it was myopia in the present invention) and cylindrical diopter (astigmatism); spherical equivalent degree = spherical diopter + 1/2 cylindrical diopter. | | | | | |

It can be seen from the data in Table 1 to Table 4 that after 6 months of continuous administration, the myopia progression of the control group was -0.36±0.13D, and the myopia progression of the test group was -0.07 + 0.15D. That was, it had a certain control effect on the increase of myopia diopter. However, due to the short administration period, there was no statistical difference between the control group and the test group. This was potentially consistent with the results of recent studies of low-concentration atropine in controlling the progression of myopia: the progression rate of myopia within 2 years in the Singapore ATOM1 placebo group was -1.20±0.69D, and the progression of myopia within 2 years in the 0.01% atropine group in the ATOM2 trial was -0.49±0.63D; that was, long-term administration of low-concentration atropine could control the progression of myopia.

However, in this study, the inventors unexpectedly found that 0.01% atropine had a therapeutic effect on transient myopia caused by near-work in a short time, there were significant differences (one-way analysis of variance) in terms of initial NITM value and attenuation time between the control group and the test group after 1 month of administration, the effective rate of drug basically reached a steady state after 90 days of administration (greater than 80.00%), and starting from 30 days, the effective rates of the test group and the control group were statistically different ($\chi^2$ test).

Example 2: Study on effects and side effects of different concentrations of atropine sulfate eye drops on NITM (not according to the claims)

1. Experimental drugs

By using 0.9% normal saline, atropine sulfate eye drops in concentrations of 0.001%, 0.005%, 0.01%, 0.02%, 0.05%, 0.1%, 0.2% were prepared, respectively, adjusted their pH to 5.5 with hydrochloric acid, and used for administration in the test groups.

2. Experimental method

120 Myopia patients aged 6 to 18 were selected. The selected patients who had eye diopter of -0.50DS to -5.00DS, astigmatism ≤ -1.0DC; initial NITM value ≥ -0.25D; normal intraocular pressure not exceeding 21mmHg were randomly divided into 8 groups, 15 patients in each group. The 7 test groups were separately dripped with the 0.001%, 0.005%, 0.01%, 0.02%, 0.05%, 0.1%, 0.2% atropine sulfate eye drops prepared with 0.9% normal saline, and the control group was dripped with 0.9% normal saline; each group was administered once a day, 1 drop per eye each time, and the aforementioned drug or 0.9% normal saline was dripped into conjunctival sac before sleep at 9 p.m. for consecutive 2 weeks. NITM detection was performed at 7 a.m. on the $0^{th}$ day, $7^{th}$ day, $14^{th}$ day, and the initial NITM value, NITM attenuation time and pupil diameter were recorded.

Optometry was carried out by a phoropter (non-cycloplegia), and pupil diameter was measured. According to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again.

3. Experimental results

The results were shown in Table 5 to Table 7 below.

Table 5: Effect of atropine sulfate eye drops in different concentrations on initial NITM value of eyes ($\bar{x}$ + S, n=15)

| Group | Observation time (days) | | |
|---|---|---|---|
| | 0 | 7 | 14 |
| Control group | 0.42±0.09 | 0.42±0.12 | 0.41±0.11 |
| 0.001% | 0.44±0.10 | 0.32±0.07 | 0.32±0.07 |
| 0.005% | 0.41±0.09 | 0.30±0.09 | 0.25±0.06 |
| 0.01% | 0.43±0.10 | 0.25±0.10 | 0.24±0.07 |
| 0.02% | 0.44±0.10 | 0.25±0.08 | 0.24±0.09 |
| 0.05% | 0.38±0.10 | 0.24±0.09 | 0.17±0.06 |
| 0.1% | 0.39±0.08 | 0.21±0.07 | 0.16±0.07 |
| 0.2% | 0.44±0.11 | 0.20±0.07 | 0.16±0.06 |

Table 6: Effect of atropine sulfate eye drops in different concentrations on NITM attenuation time of eyes ($\bar{x}$ + S, n=15)

| Group | Observation time (days) | | |
|---|---|---|---|
| | 0 | 7 | 14 |
| Control group | 136.5±7.9 | 140.6±14.1 | 136.6±9.2 |

(continued)

| Group | Observation time (days) | | |
|---|---|---|---|
| | 0 | 7 | 14 |
| 0.001% | 142.1±10.6 | 133.0±14.1 | 114.7±8.2 |
| 0.005% | 136.6±12.7 | 117.6±10.4 | 97.7±9.5 |
| 0.01% | 140.3±13.7 | 116.1±8.6 | 97.5±8.8 |
| 0.02% | 137.3±12.6 | 116.0±7.9 | 96.9±8.0 |
| 0.05% | 141.8±13.7 | 114.7±10.4 | 72.5±11.6 |
| 0.1% | 139.9±11.0 | 114.0±8.7 | 63.4±9.2 |
| 0.2% | 136.0±11.6 | 113.6±6.8 | 67.2±10.6 |

Table 7: Effect of atropine sulfate eye drops in different concentrations on pupil diameter of eyes ($\bar{x}$ + S, n=15)

| Group | Observation time (days) | | |
|---|---|---|---|
| | 0 | 7 | 14 |
| Control group | 136.5±7.9 | 3.6±0.97 | 3.7±0.73 |
| 0.001% | 142.1±10.6 | 3.9±0.48 | 4.0±0.44 |
| 0.005% | 136.6±12.7 | 4.1±0.74 | 4.1±0.70 |
| 0.01% | 140.3±13.7 | 4.2±0.68 | 4.1±0.74 |
| 0.02% | 137.3±12.6 | 4.5±0.74 | 4.8±0.71 |
| 0.05% | 141.8±13.7 | 4.7±0.72 | 4.9±0.66 |
| 0.1% | 139.9±11.0 | 4.7±0.70 | 5.1±0.98 |
| 0.2% | 136.0±11.6 | 5.2±0.48 | 5.6±1.04 |

The therapeutic effect was judged by the combination of initial NITM value and NITM attenuation time, and effectiveness was determined when there were statistical differences. Specifically, the initial NITM value was the main factor and the attenuation time was the secondary factor. The determination could be made when initial NITM value showed difference. The statistics was performed by using one-way analysis of variance, and it was considered as being of statistical difference when $P<0.05$.

From the data in Table 5, Table 6 and Table 7, it could be seen that low-concentration atropine had a therapeutic effect on NITM and had a dose-effect relationship; when the dose was increased to 0.02%, the enhancement of the therapeutic effect decreased, and the pupil diameter gradually showed a significant difference after dropping for 14 days.

<u>Example 3: Study on effect of atropine sulfate eye drops with different pH values on NITM</u> (not according to the claims)

1. Experimental drugs

By using 0.9% sodium chloride solution, 0.01% atropine sulfate eye drop was prepared, and adjusted with hydrochloric acid to pH value of 4.0, 4.5, 5.0, 5.5, 6.0, 6.5, respectively, and used for administration in the test groups.

2. Experimental method

105 Myopia patients aged 6 to 18 were selected. The selected patients who had eye diopter of -0.50DS to -5.00DS, astigmatism ≤ -1.0DC; initial NITM value ≥ -0.25D; normal intraocular pressure not exceeding 21mmHg were randomly divided into 7 groups, 15 patients in each group. The 6 test groups were separately dripped with the above prepared atropine sulfate eye drops (whose pH values were 4.0, 4.5, 5.0, 5.5, 6.0, 6.5, respectively), and the control group was dripped with 0.9% normal saline; each group was administered once a day, 1 drop per eye each time, and the aforementioned drug or 0.9% normal saline was dripped into conjunctival sac before sleep for consecutive 2 weeks.

NITM detection was performed on the $0^{th}$ day, $7^{th}$ day, $14^{th}$ day, and the initial NITM value and NITM attenuation time were recorded.

Optometry was carried out by a phoropter (non-cycloplegia). According to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again.

3. Experimental results

The results were shown in Table 8 to Table 9 below.

Table 8: Effect of atropine sulfate eye drops with different pH values on initial NITM value of eyes ($\bar{x}$ + S, n=15)

| Group | Observation time (days) | | |
|---|---|---|---|
| | 0 | 7 | 14 |
| Control group | 0.43±0.11 | 0.43±0.11 | 0.43±0.11 |
| pH4.0 | 0.45±0.10 | 0.31±0.07 | 0.31±0.08 |
| pH4.5 | 0.45±0.10 | 0.26±0.08 | 0.26±0.10 |
| pH5.0 | 0.45±0.10 | 0.24±0.09 | 0.25±0.10 |
| pH5.5 | 0.41±0.08 | 0.22±0.09 | 0.23±0.07 |
| pH6.0 | 0.46±0.09 | 0.34±0.08 | 0.35±0.07 |
| pH6.5 | 0.40±0.09 | 0.42±0.09 | 0.37±0.08 |

Table 9: Effect of atropine sulfate eye drops with different pH values on NITM attenuation time of eyes ($\bar{x}$ + S, n=15)

| Group | Observation time (days) | | |
|---|---|---|---|
| | 0 | 7 | 14 |
| Control group | 138.9±9.5 | 143.5±9.5 | 144.3±8.2 |
| pH4.0 | 142.9±10.1 | 128.8±11.3 | 125.7±12.4 |
| pH4.5 | 144.1±11.8 | 117.0±9.3 | 96.2±8.9 |
| pH5.0 | 138.7±11.8 | 116.0±11.5 | 95.6±7.9 |
| pH5.5 | 134.4±11.5 | 112.4±6.7 | 92.1±7.5 |
| pH6.0 | 142.9±11.0 | 127.0±14.1 | 112.3±7.7 |
| pH6.5 | 136.7±12.4 | 136.7±12.4 | 135.4±11.7 |

It could be seen from the data in Table 8 and Table 9 that the pH value had an impact on the effect of atropine in the treatment of NITM. The treatment effect was the best within the pH value ranging from 4.5 to 5.5. When the pH was increased to 6.0 or above, the effect would decrease, suggesting that the pH value of atropine eye drops for the treatment of NITM should preferably range from 4.5 to 5.5.

Example 4-8: Study on effect of atropine sulfate eye drops of different prescriptions on NITM (1) (not according to the claims)

1. Experimental drugs and preparation methods thereof

Table A: Prescription compositions of experimental drugs

| Component | Example 4 | Example 5 | Example 6 | Example 7 | Example 8 |
|---|---|---|---|---|---|
| Atropine sulfate | 0.005g | 0.010g | 0.020g | 0.010g | 0.020g |
| Hypromellose | -- | -- | -- | 1.000g | 1.000g |
| Sodium dihydrogen phosphate | 0.250g | 0.250g | 0.250g | 0.250g | 0.250g |
| Disodium hydrogen phosphate | 0.0025g | 0.0025g | 0.0025g | 0.0025g | 0.0025g |
| Disodium edetate | 0.010g | 0.010 g | 0.010g | 0.010g | 0.010g |
| Sodium chloride | 0.76g | 0.76g | 0.76g | 0.76g | 0.76g |
| Benzalkonium chloride | 0.010g | 0.010g | 0.010g | 0.010g | 0.010g |
| Water for injection, added to reach a volume of | 100ml | 100ml | 100ml | 100ml | 100ml |

The preparation method was as follows:

(1) 10g of 80°C to 90°C water for injection was taken, added with a prescribed amount of hypromellose (if any), supplemented with 30°C or below water for injection to 20g, stirred for dissolution to obtain a transparent solution for later use;

(2) 50g of 65°C to 75°C water for injection was taken, added with sodium dihydrogen phosphate monohydrate, disodium hydrogen phosphate, sodium chloride, disodium edetate and benzalkonium chloride in prescribed amounts in sequence for dissolution, stood to 30°C or below and added with a prescribed amount of atropine sulfate and stirred to dissolve;

(3) the hypromellose solution obtained in (1) and the solution obtained in (2) were mixed uniformly;

(4) water was supplemented to the full amount of 100g, stirred evenly, filtered and sterilized with 0.22μm filter membrane, and packaged.

They were used for administration in the test groups.

2. Experimental method

90 Myopia patients aged 6 to 18 were selected. The selected patients who had eye diopter of -0.50DS to -5.00DS, astigmatism ≤-1.0DC; initial NITM value ≥-0.25D; normal intraocular pressure not exceeding 21mmHg were randomly divided into 6 groups, 15 patients in each group. The 5 test groups were separately dripped with the above atropine eye drops (which were prepared in Examples 4 to 8), and the control group was dripped with 0.9% normal saline; each group was administered once a day, 1 drop per eye each time, and the aforementioned drug or 0.9% normal saline was dripped into conjunctival sac before sleep for consecutive 2 weeks. NITM detection was performed on the $0^{th}$ day, $7^{th}$ day, $14^{th}$ day, and the initial NITM value and NITM attenuation time were recorded.

Optometry was carried out by using a phoropter (non-cycloplegia), according to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly

(within 2s) looked at the distance mark. The long distance diopter of the right eye wearing full-correction frame glasses was measured again, and the initial NITM was recorded.

3. Experimental results

The results were shown in Table 10 to Table 11 below.

Table 10: Effect of atropine sulfate eye drops with different prescriptions on initial NITM value of eyes ($\bar{x}$ + S, n=15)

| Group | Observation time (days) | | |
|---|---|---|---|
| | 0 | 7 | 14 |
| Control group | 0.44±0.10 | 0.42±0.11 | 0.43±0.12 |
| Example 4 | 0.43±0.11 | 0.34±0.08 | 0.33±0.10 |
| Example 5 | 0.41±0.09 | 0.28±0.06 | 0.27±0.06 |
| Example 6 | 0.39±0.07 | 0.20±0.07 | 0.21±0.08 |
| Example 7 | 0.45±0.10 | 0.25±0.06 | 0.25±0.10 |
| Example 8 | 0.46±0.08 | 0.19±0.07 | 0.16±0.05 |

Table 11: Effect of atropine sulfate eye drops with different prescriptions on NITM attenuation time of eyes (X + S, n=15)

| Group | Observation time (days) | | |
|---|---|---|---|
| | 0 | 7 | 14 |
| Control group | 143.3±11.8 | 137.0±14.1 | 137.8±11.6 |
| Example 4 | 135.3±12.5 | 121.1±9.1 | 116.1±6.9 |
| Example 5 | 136.7±12.6 | 115.7±9.9 | 108.4±11.4 |
| Example 6 | 142.0±12.0 | 102.4±15.0 | 97.4±10.5 |
| Example 7 | 139.1±11.0 | 112.1±8.7 | 104.0±13.2 |
| Example 8 | 138.0±10.9 | 96.9±9.7 | 93.7±8.9 |

It could be seen from the data in Table 10 and Table 11 that after atropine was prepared as eye drops in concentrations from 0.005% to 0.02%, the atropine eye drops showed a dose-effect relationship in the treatment of NITM. After hypromellose was added in the prescriptions, the therapeutic effects tended to be enhanced, which was more obvious in high-concentration preparations; however, compared with the change of therapeutic concentration, its enhancement effect was weaker. However, it was still suggested that under the same concentration of atropine sulfate, hypromellose was preferably added to the formulation.

Example 9-13: Study on effect of atropine sulfate eye drops of different prescriptions on NITM (2)

1. Experimental drugs and preparation methods thereof

Table B: Prescription compositions of experimental drugs

| Component | Example 9 | Example 10 | Example 11 | Example 12 | Example 13 |
|---|---|---|---|---|---|
| Atropine sulfate | 0.010g | 0.020g | 0.010g | 0.020g | 0.010g |
| Hypromellose | 1.000g | 1.000g | 1.000g | 1.000g | 1.000g |
| Propylene glycol | 0.300g | 0.300g | -- | -- | -- |
| Sodium chloride | -- | -- | 0.135g | 0.135g | -- |

(continued)

| Component | Example 9 | Example 10 | Example 11 | Example 12 | Example 13 |
|---|---|---|---|---|---|
| Boric acid | 1.750g | 1.750g | 1.750g | 1.750g | 2.0g |
| Disodium edetate | 0.010g | 0.010 g | 0.010g | 0.010g | 0.010g |
| Benzalkonium chloride | 0.010g | -- | -- | 0.010g | -- |
| Water for injection, added to reach a volume of | 100ml | 100ml | 100ml | 100ml | 100ml |

Preparation method:

(1) 10g of 80°C to 90°C water for injection was taken, added with a prescribed amount of hypromellose and fully dispersed and swelled, supplemented with 30°C or below water for injection to 20g, stirred for dissolution to obtain a transparent solution for later use;

(2) 50g of 65°C to 75°C water for injection was taken, added with boric acid, propylene glycol or sodium chloride (if any), disodium edetate and benzalkonium chloride in prescribed amounts in sequence for dissolution, stood to 30°C or below and added with a prescribed amount of atropine sulfate and stirred to dissolve;

(3) the hypromellose solution obtained in (1) and the solution obtained in (2) were mixed uniformly;

(4) water was supplemented to the mixed solution obtained in (3) to reach the full amount of 100g, stirred evenly, filtered and sterilized with 0.22μm filter membrane, and packaged.

They were used for administration in the test groups.

2. Experimental method

90 Myopia patients aged 6 to 18 were selected. The selected patients who had eye diopter of -0.50DS to -5.00DS, astigmatism ≤-1.0DC; initial NITM value ≥-0.25D; normal intraocular pressure not exceeding 21mmHg were randomly divided into 6 groups, 15 patients in each group. The 5 test groups were separately dripped with the above atropine eye drops (which were prepared in Examples 9 to 13), and the control group was dripped with 0.9% normal saline; each group was administered once a day, 1 drop per eye each time, and the aforementioned drug or 0.9% normal saline was dripped into conjunctival sac before sleep for consecutive 2 weeks. NITM detection was performed on the $0^{th}$ day, $7^{th}$ day, $14^{th}$ day, and the initial NITM value, NITM attenuation time and pupil diameter before the detection of NITM were recorded.

Optometry was carried out by using a phoropter (non-cycloplegia) and the pupil diameter was measured, according to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again.

3. Experimental results

The results were shown in Table 12 to Table 14 below.

Table 12: Effect of atropine sulfate eye drops with different prescriptions on initial NITM value of eyes ($\bar{x}$ + S, n=15)

| Group | Observation time (days) | | |
|---|---|---|---|
| | 0 | 7 | 14 |
| Control group | 0.44±0.14 | 0.45±0.10 | 0.46±0.09 |
| Example 9 | 0.44±0.10 | 0.22±0.07 | 0.21±0.08 |
| Example 10 | 0.42±0.10 | 0.14±0.05 | 0.13±0.07 |
| Example 11 | 0.44±0.12 | 0.25±0.08 | 0.23±0.08 |
| Example 12 | 0.41±0.10 | 0.18±0.07 | 0.15±0.05 |
| Example 13 | 0.46±0.09 | 0.20±0.06 | 0.20±0.07 |

Table 13: Effect of atropine sulfate eye drops with different prescriptions on NITM attenuation time of eyes (X + S, n=15)

| Group | Observation time (days) | | |
|---|---|---|---|
| | 0 | 7 | 14 |
| Control group | 137.2±12.6 | 142.7±11.8 | 140.0±11.3 |
| Example 9 | 140.0±12.4 | 107.2.1±12.1 | 98.2±12.2 |
| Example 10 | 136.7±9.3 | 93.8±8.1 | 88.9±8.3 |
| Example 11 | 140.3±12.3 | 113.1±7.7 | 100.3±12.9 |
| Example 12 | 141.3±11.7 | 96.1±9.8 | 91.2±7.7 |
| Example 13 | 142.5±12.9 | 100.7±8.4 | 95.1±8.0 |

Table 14: Effect of atropine sulfate eye drops with different prescriptions on pupil diameter of eyes (X + S, n=15)

| Group | Observation time (days) | | |
|---|---|---|---|
| | 0 | 7 | 14 |
| Control group | 3.6±0.9 | 3.6±1.0 | 3.3±0.9 |
| Example 9 | 3.6±0.9 | 4.4±0.7 | 4.1±0.8 |
| Example 10 | 3.8±0.8 | 5.0±1.0 | 4.8±0.9 |
| Example 11 | 3.8±0.9 | 4.3±0.8 | 4.3±0.9 |
| Example 12 | 3.2±0.9 | 5.2±1.0 | 5.0±0.8 |
| Example 13 | 3.4±0.8 | 4.2±0.8 | 3.9±0.6 |

The comparison of the data in Table 12 and Table 13 showed that when boric acid was used as the buffer system, the therapeutic effect of atropine eye drops on NITM was better than that of the phosphate buffer system. When polyol was used as the osmotic pressure regulator, the therapeutic effect was slightly better than that of the inorganic salt osmotic pressure regulator, but the inventors unexpectedly discovered that when only boric acid was used as the pH regulating agent and used to adjust the osmotic pressure at the same time, much better therapeutic effect was achieved.

It could be seen from the data in Table 14 that the boric acid buffer salt system could not only improve the therapeutic effect on NITM, but also had low mydriatic side effect, especially when boric acid was used alone (Example 13), the weakest mydriatic effect was achieved.

Examples 14-18: Study on effect of atropine sulfate eye drops of different prescriptions on NITM (3) (Example 18 is not according to the claims)

1. Experimental drugs and preparation methods thereof

Table C: Prescription compositions of experimental drugs

| Component | Example 14 | Example 15 | Example 16 | Example 17 | Example 18 |
|---|---|---|---|---|---|
| Atropine sulfate | 0.010g | 0.010g | 0.010g | 0.010g | 0.010g |
| Hypromellose | 0.800g | 0.500g | 1.000g | 1.200g | 1.000g |
| Sodium hyaluronate | 0.02 g | 0.05g | -- | -- | -- |
| Boric acid | 2.00g | 2.00g | 1.750g | 2.0g | 1.750g |
| Disodium edetate | 0.005g | 0.010g | 0.050g | 0.010g | 0.100g |
| Benzalkonium chloride | -- | 0.010g | -- | 0.010g | -- |
| Hydrochloric acid or sodium hydroxide, added to adjust pH to | 4.5 | 5.0 | 5.5 | 5.0 | 6.0 |
| Water for injection, added to reach a volume of | 100ml | 100ml | 100ml | 100ml | 100ml |

Preparation method:

(1) 10g of 80°C to 90°C water for injection was taken, added with a prescribed amount of hypromellose or sodium hyaluronate (if any) and fully dispersed and swelled, supplemented with 30°C or below water for injection to 20g, stirred for dissolution to obtain a transparent solution for later use;

(2) 50g of 65°C to 75°C water for injection was taken, added with boric acid, disodium edetate and benzalkonium chloride (if any) in prescribed amounts in sequence for dissolution, stood to 30°C or below and added with a prescribed amount of atropine sulfate and stirred to dissolve;

(3) the solution obtained in (1) and the solution obtained in (2) were mixed uniformly;

(4) water was supplemented to the mixed solution obtained in (3) to reach the full amount of 100g, stirred evenly, filtered and sterilized with 0.22μm filter membrane, and packaged.

They were used for administration in the test groups.

2. Experimental method

90 Myopia patients aged 6 to 18 were selected. The selected patients who had eye diopter of -0.50DS to -5.00DS, astigmatism ≤-1.0DC; initial NITM value ≥-0.25D; normal intraocular pressure not exceeding 21mmHg were randomly divided into 6 groups, 15 patients in each group. The 5 test groups were separately dripped with the above atropine eye drops (which were prepared in Examples 14 to 18), and the control group was dripped with 0.9% normal saline; each group was administered once a day, 1 drop per eye each time, and the aforementioned drug or 0.9% normal saline was dripped into conjunctival sac before sleep for consecutive 2 weeks. NITM detection was performed on the $0^{th}$ day, $7^{th}$ day, $14^{th}$ day, and the initial NITM value and attenuation time were recorded.

Optometry was carried out by using a phoropter (non-cycloplegia) and the pupil diameter was measured, according to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was

recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again, and the initial NITM was recorded.

3. Experimental results

The results were shown in Table 15 to Table 16 below.

Table 15: Effect of atropine sulfate eye drops with different prescriptions on initial NITM value of eyes ($\bar{x}$ + S, n=15)

| Group | Observation time (days) | | |
|---|---|---|---|
| | 0 | 7 | 14 |
| Control group | 0.46±0.11 | 0.42±0.11 | 0.40±0.09 |
| Example 14 | 0.45±0.11 | 0.26±0.09 | 0.23±0.07 |
| Example 15 | 0.44±0.09 | 0.23±0.07 | 0.20±0.07 |
| Example 16 | 0.43±0.11 | 0.22±0.07 | 0.20±0.08 |
| Example 17 | 0.44±0.10 | 0.23±0.07 | 0.24±0.04 |
| Example 18 | 0.45±0.09 | 0.36±0.09 | 0.34±0.07 |

Table 16: Effect of atropine sulfate eye drops with different prescriptions on NITM attenuation time of eyes (X + S, n=15)

| Group | Observation time (days) | | |
|---|---|---|---|
| | 0 | 7 | 14 |
| Control group | 143.3±14.2 | 139.2±11.9 | 141.9±13.2 |
| Example 14 | 137.1±13.6 | 108.7±7.2 | 91.5±10.5 |
| Example 15 | 139.3±9.1 | 106.8±12.1 | 86.8±8.0 |
| Example 16 | 143.1±10.1 | 105.9±12.6 | 83.9±102 |
| Example 17 | 142.6±10.1 | 106.5±15.4 | 92.5±10.6 |
| Example 18 | 138.3±13.6 | 126.4±13.9 | 107.1±13.5 |

The results show that the pharmaceutical compositions of the present invention could effectively treat and/or prevent NITM in patients with myopia. In addition, the results also showed that the effects of the pharmaceutical compositions prepared in Examples 14 to 17 were similar, while the effect of the pharmaceutical composition of Example 18 was relatively poor, so the preferred pH was 6.0 or less.

Examples 19-23: Study on effect of atropine sulfate eye drops of different prescriptions on NITM (4)

1. Experimental drugs and preparation methods thereof

The experimental drugs used in Examples 19-23 were exactly the same as those used in the previous Examples 14-18. (Example 18 is not according to the claims)
They were used for administration in the test group.

2. Experimental method

90 Persons aged 6 to 18 with normal eyesight were selected, who had initial NITM value≥ -0.20D; normal intraocular pressure not exceeding 21mmHg were randomly divided into 6 groups, 15 persons in each group. The 5 test groups were separately dripped with the above atropine eye drops (which were prepared in Examples 14 to 18), and the control group was dripped with 0.9% normal saline; each group was administered once a day, 1 drop per eye each

time, and dripped into conjunctival sac before sleep for consecutive 2 weeks. NITM detection was performed on the $0^{th}$ day, $7^{th}$ day, $14^{th}$ day, and the initial NITM value and attenuation time were recorded.

Optometry was carried out by using a phoropter (non-cycloplegia) and the pupil diameter was measured, according to the optometry results, full-correction frame glasses were worn to correct the distance vision to the best corrected visual acuity, and the spherical equivalent degree was obtained. A rest for 10 minutes in a complete dark room was taken to relax any possible adjustments. Then, an infrared auto-refractor (WAM-5500, Grand Seiko, Japan) was used, both eyes looked at the 5m distance mark, the long distance diopter of right eye was measured, and the data was recorded. Then near-work was started, reading was performed with both eyes for the near-work, the reading content was simplified Chinese text of 12pt font, the reading distance was 35 to 40 cm, and the reading time was 1h. All subjects should pay attention to the text and keep the text clear, and should not be distracted during the procedure. After finishing the near-work, the subject quickly (within 2s) looked at the distance mark. The long distance diopter of the right eye wearing full-correction frame glasses was measured again, and the initial NITM was recorded.

3. Experimental results

The results were shown in Table 17 to Table 18 below.

Table 17: Effect of atropine sulfate eye drops with different prescriptions on initial NITM value of emmetropic eyes ($\bar{x}$ + S, n=15)

| Group | Observation time (days) | | |
|---|---|---|---|
| | 0 | 7 | 14 |
| Control group | 0.34±0.07 | 0.32±0.08 | 0.31±0.06 |
| Example 14 | 0.31±0.06 | 0.26±0.10 | 0.16±0.06 |
| Example 15 | 0.33±0.06 | 0.24±0.09 | 0.15±0.06 |
| Example 16 | 0.32±0.07 | 0.22±0.09 | 0.15±0.07 |
| Example 17 | 0.33±0.06 | 0.26±0.09 | 0.16±0.07 |
| Example 18 | 0.34±0.06 | 0.29±0.05 | 0.22±0.08 |

Table 18: Effect of atropine sulfate eye drops with different prescriptions on NITM attenuation time of emmetropic eyes ($\bar{x}$ + S, n=15)

| Group | Observation time (days) | | |
|---|---|---|---|
| | 0 | 7 | 14 |
| Control group | 70.7±10.2 | 72.3±11.4 | 67.5±13.3 |
| Example 14 | 71.0±13.5 | 51.8±14.6 | 41.4±5.6 |
| Example 15 | 69.5±11.2 | 47.4±10.9 | 38.5±6.3 |
| Example 16 | 65.7±9.6 | 45.3±12.6 | 37.5±5.0 |
| Example 17 | 66.7±8.7 | 49.1±11.2 | 42.0±4.6 |
| Example 18 | 67.7±12.0 | 60.5±11.5 | 55.5±10.1 |

The results showed that the pharmaceutical compositions of the present invention could effectively treat and/or prevent NITM in subjects with normal eyesight. In addition, the results also showed that the effects of the pharmaceutical compositions prepared in Examples 14-17 were similar, and the effect of the pharmaceutical composition of Example 18 was relatively poor, so the preferred pH was 6.0 or less.

## Claims

1. A pharmaceutical composition, comprising atropine or a pharmaceutically acceptable salt thereof in an amount of 0.001% weight to 0.2% weight, and one or more pharmaceutically acceptable excipients;

wherein,

the pharmaceutical composition has a pH value of 4.5 to 5.5;
the pharmaceutical composition comprises a pH adjusting agent in an amount of 0.05% weight to 5% weight, and the pH adjusting agent is one or more selected from a group consisting of boric acid and borate.

**2.** The pharmaceutical composition according to claim 1, which is an ophthalmic preparation, such as an ophthalmic liquid preparation (e.g., eye drop, eye wash or intraocular injection solution), ophthalmic semi-solid preparation (e.g., eye ointment, ophthalmic cream or ophthalmic gel), or ophthalmic solid preparation (e.g., eye film, eye pill or intraocular insert); optionally, the ophthalmic liquid preparation may be packaged in a solid form, in which a solvent is provided separately, and a solution or suspension is prepared before use.

**3.** The pharmaceutical composition according to claim 2, wherein the atropine or a pharmaceutically acceptable salt thereof has a concentration or content of 0.001% weight to 0.1% weight, preferably 0.005% weight to 0.05% weight, more preferably 0.005% weight to 0.02% weight, more preferably 0.005% weight to 0.015% weight, particularly preferably 0.01% weight; preferably, the pharmaceutically acceptable salt of atropine is atropine sulfate.

**4.** The pharmaceutical composition according to any one of claims 1 to 3, wherein:

the pH adjusting agent has a content of 0.5% weight to 5% weight, more preferably 1% weight to 3% weight, further preferably 1.5% weight to 2.5% weight, particularly preferably 1.75% weight to 2.0% weight;
preferably, the borate salt is sodium tetraborate.

**5.** The pharmaceutical composition according to any one of claims 1 to 3, wherein the pharmaceutical composition comprises boric acid in an amount of 0.5% weight to 3% weight, 1% weight to 3% weight, 1.5% weight to 2.5% weight, 1.75% weight to 2.25% weight or 1.75% weight to 2% weight.

**6.** The pharmaceutical composition according to any one of claims 1 to 3, wherein the pharmaceutical composition further comprises any one or more selected from the following items 1) to 5):

1) a thickener, and the thickener is any one or more selected from a group consisting of hypromellose, sodium carboxymethyl cellulose and sodium hyaluronate; preferably, the thickener has a content of 0.01 % weight to 5% weight, preferably 0.5% weight to 3% weight, more preferably 0.5% weight to 1.5% weight, particularly preferably 0.8% weight to 1.2% weight;
2) an osmotic pressure regulator, for example, any one or more selected from a group consisting of glycerin, mannitol, propylene glycol, sodium chloride and potassium chloride;
3) a preservative, for example, any one or more selected from a group consisting of benzalkonium chloride, paraben bacteriostatic agents, and polyquaterniums;
4) a stabilizer, for example, any one or more selected from a group consisting of disodium edetate and calcium sodium edetate;
5) an appropriate amount of water.

**7.** The pharmaceutical composition according to any one of claims 1 to 3, wherein the ingredients and contents thereof in the pharmaceutical composition are selected from any one of the following groups (1) to (6):

(1)

| | |
|---|---|
| Atropine sulfate | 0.01 parts by weight |
| Hypromellose | 0.5 to 1.2 parts by weight |
| Sodium hyaluronate | 0 to 0.05 parts by weight |
| Boric acid | 1.5 to 2.0 parts by weight |
| Disodium edetate | 0.005 to 0.1 parts by weight |
| Benzalkonium chloride | 0 to 0.01 parts by weight |

Hydrochloric acid or sodium hydroxide, added to adjust pH to 4.5 to 5.5 Water for injection, added to reach 100 parts by weight;
(2)

| | |
|---|---|
| Atropine sulfate | 0.01 parts by weight |
| Hypromellose | 0.8 to 1.0 parts by weight |
| Sodium hyaluronate | 0.02 to 0.05 parts by weight |
| Boric acid | 1.75 to 2.0 parts by weight |
| Disodium edetate | 0.01 to 0.05 parts by weight |
| Benzalkonium chloride | 0 to 0.01 parts by weight |

Hydrochloric acid or sodium hydroxide, added to adjust pH to 4.5 to 5.5 Water for injection, added to reach 100 parts by weight;
(3)

| | |
|---|---|
| Atropine sulfate | 0.010 parts by weight |
| Hypromellose | 0.800 parts by weight |
| Sodium hyaluronate | 0.02 parts by weight |
| Boric acid | 2.00 parts by weight |
| Disodium edetate | 0.005 parts by weight |
| Benzalkonium chloride | 0 parts by weight |

Hydrochloric acid or sodium hydroxide, added to adjust pH to 4.5 Water for injection, added to reach 100 parts by weight;
(4)

| | |
|---|---|
| Atropine sulfate | 0.010 parts by weight |
| Hypromellose | 0.500 parts by weight |
| Sodium hyaluronate | 0.05 parts by weight |
| Boric acid | 2.00 parts by weight |
| Disodium edetate | 0.010 parts by weight |
| Benzalkonium chloride | 0.010 parts by weight |

Hydrochloric acid or sodium hydroxide, added to adjust pH to 5.0 Water for injection, added to reach 100 parts by weight;
(5)

| | |
|---|---|
| Atropine sulfate | 0.010 parts by weight |
| Hypromellose | 1.000 parts by weight |
| Sodium hyaluronate | 0 parts by weight |
| Boric acid | 1.750 parts by weight |
| Disodium edetate | 0.050 parts by weight |
| Benzalkonium chloride | 0 parts by weight |

Hydrochloric acid or sodium hydroxide, added to adjust pH to 5.5 Water for injection, added to reach 100 parts by weight;
(6)

| | |
|---|---|
| Atropine sulfate | 0.010 parts by weight |
| Hypromellose | 1.200 parts by weight |
| Sodium hyaluronate | 0 parts by weight |
| Boric acid | 2.0 parts by weight |
| Disodium edetate | 0.010 parts by weight |
| Benzalkonium chloride | 0.010 parts by weight |

Hydrochloric acid or sodium hydroxide, added to adjust pH to 5.0 Water for injection, added to reach 100 parts

by weight.

8. The pharmaceutical composition according to any one of claims 1 to 7 for use in treatment and/or prevention of NITM.

9. The pharmaceutical composition for the use according to claim 8, wherein the pharmaceutical composition is an ophthalmic preparation, preferably an eye drop.

10. The pharmaceutical composition for the use according to claim 9, wherein the eye drop is administered once a day or every other day, with 1 to 2 drops each time; preferably, the eye drop is dripped into a conjunctival sac.

11. The pharmaceutical composition for the use according to claim 9 or 10, wherein the eye drop is administered to a subject who is a person using eyes in near distance or a person susceptible to myopia;

    preferably, the person using eyes in near distance is a myopia patient or a non-myopia patient;
    preferably, the person susceptible to myopia is a person aged 6 to 18.

## Patentansprüche

1. Pharmazeutische Zusammensetzung, umfassend Atropin oder ein pharmazeutisch verträgliches Salz davon in einer Menge von 0,001 Gewichts-% bis 0,2 Gewichts-% und ein oder mehrere pharmazeutisch akzeptable Hilfsstoffe; worin,

    die pharmazeutische Zusammensetzung einen pH-Wert von 4,5 bis 5,5 aufweist;
    die pharmazeutische Zusammensetzung ein Mittel zur pH-Einstellung in einer Menge von 0,05 Gewichts-% bis 5 Gewichts-% aufweist, und das Mittel zur pH-Einstellung ist ein Element oder sind mehrere Elemente, das/die aus der Gruppe, bestehend aus Borsäure und Borat, ausgewählt wird / werden.

2. Pharmazeutische Zusammensetzung nach Anspruch 1, bei der es sich um ein Augenpräparat handelt, wie ein flüssiges Augenpräparat (z. B. Augentropfen, Augenspülmittel oder intraokulare Einspritzlösung), ein halbfestes Augenpräparat (z. B. Augensalbe, Augencreme oder Augengel) oder ein festes Augenpräparat (z. B. Augenfilm, Augentablette oder Augeneinsatz), wobei optional das flüssige Augenpräparat in einer festen Form verpackt sein kann, zu der ein Lösungsmittel separat bereitgestellt wird und vor der Verwendung eine Lösung oder Suspension hergestellt wird.

3. Pharmazeutische Zusammensetzung nach Anspruch 2, wobei das Atropin oder ein pharmazeutisch verträgliches Salz davon eine Konzentration bzw. einen Gehalt von 0,001 Gewichts-% bis 0,1 Gewichts-%, vorzugsweise 0,005 Gewichts-% bis 0,05 Gewichts-%, bevorzugter 0,005 Gewichts-% bis 0,02 Gewichts-%, bevorzugter 0,005 Gewichts-% bis 0,015 Gewichts-%, besonders bevorzugt 0,01 Gewichts-% aufweist; vorzugsweise besteht das pharmazeutisch verträgliche Atropinsalz aus Atropinsulfat.

4. Pharmazeutische Zusammensetzung nach irgendeinem der Ansprüche 1 bis 3, wobei:
   das Mittel zur pH-Einstellung einen Gehalt von 0,5 Gewichts-% bis 5 Gewichts-%, bevorzugter 1 Gewichts-% bis 3 Gewichts-%, weiter bevorzugt 1,5 Gewichts-% bis 2,5 Gewichts-%, besonders bevorzugt 1,75 Gewichts-% bis 2,0 Gewichts-% hat; vorzugsweise besteht das Boratsalz aus Natriumtetraborat.

5. Pharmazeutische Zusammensetzung nach irgendeinem der Ansprüche 1 bis 3, wobei die pharmazeutische Zusammensetzung Borsäure in einer Menge von 0,5 Gewichts-% bis 3 Gewichts-%, 1 Gewichts-% bis 3 Gewichts-%, 1,5 % Gewichts-% bis 2,5 Gewichts-%, 1,75 Gewichts-% bis 2,25 Gewichts-% oder 1,75 Gewichts-% bis 2 Gewichts-% enthält.

6. Pharmazeutische Zusammensetzung nach irgendeinem der Ansprüche 1 bis 3, wobei die pharmazeutische Zusammensetzung außerdem irgendeinem oder mehrere der folgenden Elemente 1) bis 5) aufweist:

    1) ein Verdickungsmittel, und das Verdickungsmittel besteht aus irgendeinem oder mehreren Elementen, ausgewählt aus einer Gruppe, die aus Hypromellose, Natriumcarboxymethyl-Cellulose und Natriumhyaluronat besteht, vorzugsweise hat das Verdickungsmittel einen Gehalt von 0,01 Gewichts-% bis 5 Gewichts-%, vorzugsweise 0,5 Gewichts-% bis 3 Gewichts-%, bevorzugter 0,5 Gewichts-% bis 1,5 Gewichts-%, besonders bevorzugt

0,8 Gewichts-% bis 1,2 Gewichts-%,
2) einen osmotischen Druckregulierer, zum Beispiel irgendeinem oder mehreren Elementen, ausgewählt aus einer Gruppe, die aus Glycerin, Mannitol, Propylenglykol, Natriumchlorid und Kaliumchlorid besteht,
3) ein Konservierungsmittel, zum Beispiel irgendeinem oder mehreren Elementen, ausgewählt aus einer Gruppe, die aus Benzalkoniumchlorid, bakterienhemmenden Parabenmitteln und Polyquatemiumen besteht,
4) einen Stabilisator, zum Beispiel irgendeinem oder mehreren Elementen, ausgewählt aus der Gruppe, die aus Dinatrium-Edetat und Calcium-Natriumedetat besteht,
5) eine angemessene Menge an Wasser.

7. Pharmazeutische Zusammensetzung nach irgendeinem der Ansprüche 1 bis 3, wobei die Inhaltsstoffe und deren Gehalte in der pharmazeutischen Zusammensetzung aus irgendeinem der folgenden Gruppen (1) bis (6) ausgewählt werden:

(1)

| | |
|---|---|
| Atropinsulfat | 0,01 Gewichtsteile |
| Hypromellose | 0,5 bis 1,2 Gewichtsteile |
| Natriumhyaluronat | 0 bis 0,05 Gewichtsteile |
| Borsäure | 1,5 bis 2,0 Gewichtsteile |
| Dinatriumedetat | 0,005 bis 0,1 Gewichtsteile |
| Benzalkoniumchlorid | 0 bis 0,01 Gewichtsteile |

Salzsäure oder Natriumhydroxid werden hinzugefügt, um den pH-Wert auf 4,5 bis 5,5 einzustellen;
Wasser zur Einspritzung wird zugesetzt, um 100 Gewichtsteile zu erreichen;

(2)

| | |
|---|---|
| Atropinsulfat | 0,01 Gewichtsteile |
| Hypromellose | 0,8 bis 1,0 Gewichtsteile |
| Natriumhyaluronat | 0,02 bis 0,05 Gewichtsteile |
| Borsäure | 1,75 bis 2,0 Gewichtsteile |
| Dinatriumedetat | 0,01 bis 0,05 Gewichtsteile |
| Benzalkoniumchlorid | 0 bis 0,01 Gewichtsteile |

Salzsäure oder Natriumhydroxid werden hinzugefügt, um den pH-Wert auf 4,5 bis 5,5 einzustellen;
Wasser zur Einspritzung wird zugesetzt, um 100 Gewichtsteile zu erreichen,

(3)

| | |
|---|---|
| Atropinsulfat | 0,010 Gewichtsteile |
| Hypromellose | 0,800 Gewichtsteile |
| Natriumhyaluronat | 0,02 Gewichtsteile |
| Borsäure | 2,00 Gewichtsteile |
| Dinatriumedetat | 0,005 Gewichtsteile |
| Benzalkoniumchlorid | 0 Gewichtsteile |

Salzsäure oder Natriumhydroxid werden hinzugefügt, um den pH-Wert auf 4,5 einzustellen;
Wasser zur Einspritzung wird zugesetzt, um 100 Gewichtsteile zu erreichen,

(4)

| | |
|---|---|
| Atropinsulfat | 0,010 Gewichtsteile |
| Hypromellose | 0,500 Gewichtsteile |
| Natriumhyaluronat | 0,05 Gewichtsteile |

(fortgesetzt)

| | |
|---|---|
| Borsäure | 2,00 Gewichtsteile |
| Dinatriumedetat | 0,010 Gewichtsteile |
| Benzalkoniumchlorid | 0,010 Gewichtsteile |

Salzsäure oder Natriumhydroxid werden hinzugefügt, um den pH-Wert auf 5,0 einzustellen
Wasser zur Einspritzung wird zugesetzt, um 100 Gewichtsteile zu erreichen;

(5)

| | |
|---|---|
| Atropinsulfat | 0,010 Gewichtsteile |
| Hypromellose | 1,000 Gewichtsteile |
| Natriumhyaluronat | 0 Gewichtsteile |
| Borsäure | 1,750 Gewichtsteile |
| Dinatriumedetat | 0,050 Gewichtsteile |
| Benzalkoniumchlorid | 0 Gewichtsteile |

Salzsäure oder Natriumhydroxid werden hinzugefügt, um den pH-Wert auf 5,5 einzustellen;
Wasser zur Einspritzung wird zugesetzt, um 100 Gewichtsteile zu erreichen,

(6)

| | |
|---|---|
| Atropinsulfat | 0,010 Gewichtsteile |
| Hypromellose | 1,200 Gewichtsteile |
| Natriumhyaluronat | 0 Gewichtsteile |
| Borsäure | 2,0 Gewichtsteile |
| Dinatriumedetat | 0,010 Gewichtsteile |
| Benzalkoniumchlorid | 0,010 Gewichtsteile |

Salzsäure oder Natriumhydroxid werden hinzugefügt, um den pH-Wert auf 5,0 einzustellen;
Wasser zur Einspritzung wird zugesetzt, um 100 Gewichtsteile zu erreichen.

**8.** Pharmazeutische Zusammensetzung nach irgendeinem der Ansprüche 1 bis 7 zur Verwendung bei der Behandlung und/oder Vorbeugung von NITM.

**9.** Pharmazeutische Zusammensetzung zur Verwendung nach Anspruch 8, wobei die es sich bei der pharmazeutischen Zusammensetzung um ein Augenpräparat handelt, vorzugsweise um Augentropfen.

**10.** Pharmazeutische Zusammensetzung zur Verwendung nach Anspruch 9, wobei die Augentropfen einmal täglich oder jeden zweiten Tag mit jeweils 1 bis 2 Tropfen verabreicht werden, vorzugsweise wird der Augentropfen in einem Bindehautsack getropft.

**11.** Pharmazeutische Zusammensetzung zur Verwendung nach Anspruch 9 oder 10,

wobei der Augentropfen einem Probanden verabreicht wird, bei dem es sich um eine Person handelt, die die Augen in der Nähe benutzt, oder um eine Person, die anfällig für Kurzsichtigkeit ist;
vorzugsweise handelt es sich bei der Person, die die Augen in der Nähe nutzt, um einem Patienten mit Kurzsichtigkeit oder einem Patienten ohne Kurzsichtigkeit; vorzugsweise handelt es sich bei der Person, die anfällig für Kurzsichtigkeit ist, um eine Person im Alter von 6 bis 18 Jahren.

## Revendications

**1.** Composition pharmaceutique comprenant de l’atropine ou un sel pharmaceutiquement acceptable de celle-ci en une quantité de 0,001 % en poids à 0,2 % en poids, et un ou plusieurs excipients pharmaceutiquement acceptables ;

dans laquelle

la composition pharmaceutique a un pH de 4,5 à 5,5 ;
la composition pharmaceutique comprend un agent d'ajustement du pH en une quantité de 0,05 % en poids à 5 % en poids, et l'agent d'ajustement du pH est un ou plusieurs choisis dans le groupe constitué par l'acide borique et un borate.

**2.** Composition pharmaceutique selon la revendication 1, qui est une préparation ophtalmique, telle qu'une préparation liquide ophtalmique (par exemple une goutte oculaire, un lavage oculaire ou une solution pour injection intraoculaire), une préparation semi-solide ophtalmique (par exemple une pommade oculaire, une crème ophtalmique ou un gel ophtalmique), ou une préparation solide ophtalmique (par exemple un film oculaire, une pilule oculaire ou un pellet intraoculaire) ; éventuellement la préparation liquide ophtalmique peut être conditionnée sous une forme solide, dans laquelle un solvant est fourni séparément, et une solution ou suspension est préparée avant utilisation.

**3.** Composition pharmaceutique selon la revendication 2, dans laquelle l'atropine ou un sel pharmaceutiquement acceptable de celle-ci a une concentration ou teneur de 0,001 % en poids à 0,1 % en poids, de préférence de 0,005 % en poids à 0,05 % en poids, mieux encore de 0,005 % en poids à 0,02 % en poids, mieux encore de 0,005 % en poids à 0,015 % en poids, plus particulièrement de 0,01 % en poids ; de préférence le sel pharmaceutiquement acceptable d'atropine est le sulfate d'atropine.

**4.** Composition pharmaceutique selon l'une quelconque des revendications 1 à 3, dans laquelle :

l'agent d'ajustement du pH a une teneur de 0,5 % en poids à 5 % en poids, mieux encore de 1 % en poids à 3 % en poids, plus particulièrement de 1,5 % en poids à 2,5 % en poids, tout spécialement de 1,75 % en poids à 2,0 % en poids ;
de préférence, le sel borate est le tétraborate de sodium.

**5.** Composition pharmaceutique selon l'une quelconque des revendications 1 à 3, dans laquelle la composition pharmaceutique comprend de l'acide borique en une quantité de 0,5 % en poids à 3 % en poids, de 1 % en poids à 3 % en poids, de 1,5 % en poids à 2,5 % en poids, de 1,75 % en poids à 2,25 % en poids, ou de 1,75 % en poids à 2 % en poids.

**6.** Composition pharmaceutique selon l'une quelconque des revendications 1 à 3, dans laquelle la composition pharmaceutique comprend en outre l'un quelconque ou plusieurs choisis parmi les points 1) à 5) suivants :

1) un épaississant, et l'épaississant est l'un quelconque ou plusieurs choisis dans le groupe constitué par l'hypromellose, la carboxyméthylcellulose sodique et le hyaluronate de sodium ; de préférence l'épaississant a une teneur de 0,01 % en poids à 5 % en poids, de préférence de 0,5 % en poids à 3 % en poids, mieux encore de 0,5 % en poids à 1,5 % en poids, plus particulièrement de 0,8 % en poids à 1,2 % en poids ;
2) un régulateur de pression osmotique, par exemple l'un quelconque ou plusieurs choisis dans le groupe constitué par le glycérol, le mannitol, le propylèneglycol, le chlorure de sodium et le chlorure de potassium ;
3) un conservateur, par exemple l'un quelconque ou plusieurs choisis dans le groupe constitué par le chlorure de benzalkonium, les agents bactériostatiques de type paraben, et les polyquaterniums ;
4) un stabilisant, par exemple l'un quelconque ou plusieurs choisis dans le groupe constitué par l'édétate disodique et l'édétate de calcium et de sodium ;
5) une quantité appropriée d'eau.

**7.** Composition pharmaceutique selon l'une quelconque des revendications 1 à 3, dans laquelle les ingrédients et leurs teneurs dans la composition pharmaceutique sont choisis parmi n'importe lesquels des groupes (1) à (6) suivants :

(1)

| | |
|---|---|
| Sulfate d'atropine | 0,01 partie en poids |
| Hypromellose | 0,5 à 1,2 parties en poids |
| Hyaluronate de sodium | 0 à 0,05 partie en poids |
| Acide borique | 1,5 à 2,0 parties en poids |
| Edétate disodique | 0,005 à 0,1 partie en poids |

(suite)

| | |
|---|---|
| Chlorure de benzalkonium | 0 à 0,01 partie en poids |

Acide chlorhydrique ou hydroxyde de sodium, ajouté pour ajuster le pH à 4,5-5,5 Eau pour injection, ajoutée pour atteindre 100 parties en poids ;
(2)

| | |
|---|---|
| Sulfate d'atropine | 0,01 partie en poids |
| Hypromellose | 0,8 à 1,0 partie en poids |
| Hyaluronate de sodium | 0,02 à 0,05 partie en poids |
| Acide borique | 1,75 à 2,0 parties en poids |
| Edétate disodique | 0,01 à 0,05 partie en poids |
| Chlorure de benzalkonium | 0 à 0,01 partie en poids |

Acide chlorhydrique ou hydroxyde de sodium, ajouté pour ajuster le pH à 4,5-5,5 Eau pour injection, ajoutée pour atteindre 100 parties en poids ;
(3)

| | |
|---|---|
| Sulfate d'atropine | 0,010 partie en poids |
| Hypromellose | 0,800 partie en poids |
| Hyaluronate de sodium | 0,02 partie en poids |
| Acide borique | 2,00 parties en poids |
| Edétate disodique | 0,005 partie en poids |
| Chlorure de benzalkonium | 0 partie en poids |

Acide chlorhydrique ou hydroxyde de sodium, ajouté pour ajuster le pH à 4,5 Eau pour injection, ajoutée pour atteindre 100 parties en poids ;
(4)

| | |
|---|---|
| Sulfate d'atropine | 0,010 partie en poids |
| Hypromellose | 0,500 partie en poids |
| Hyaluronate de sodium | 0,05 partie en poids |
| Acide borique | 2,00 parties en poids |
| Edétate disodique | 0,010 partie en poids |
| Chlorure de benzalkonium | 0,010 partie en poids |

Acide chlorhydrique ou hydroxyde de sodium, ajouté pour ajuster le pH à 5,0 Eau pour injection, ajoutée pour atteindre 100 parties en poids ;
(5)

| | |
|---|---|
| Sulfate d'atropine | 0,010 partie en poids |
| Hypromellose | 1,000 partie en poids |
| Hyaluronate de sodium | 0 partie en poids |
| Acide borique | 1,750 parties en poids |
| Edétate disodique | 0,050 partie en poids |
| Chlorure de benzalkonium | 0 partie en poids |

Acide chlorhydrique ou hydroxyde de sodium, ajouté pour ajuster le pH à 5,5 Eau pour injection, ajoutée pour atteindre 100 parties en poids ;
(6)

| | |
|---|---|
| Sulfate d'atropine | 0,010 partie en poids |
| Hypromellose | 1,200 partie en poids |

(suite)

| | |
|---|---|
| Hyaluronate de sodium | 0 partie en poids |
| Acide borique | 2,0 parties en poids |
| Edétate disodique | 0,010 partie en poids |
| Chlorure de benzalkonium | 0,010 partie en poids |

Acide chlorhydrique ou hydroxyde de sodium, ajouté pour ajuster le pH à 5,0 Eau pour injection, ajoutée pour atteindre 100 parties en poids.

**8.** Composition pharmaceutique selon l'une quelconque des revendications 1 à 7, pour une utilisation dans le traitement et/ou la prévention de la Myopie transitoire induite par le travail de près (NITM).

**9.** Composition pharmaceutique pour une utilisation selon la revendication 8, dans laquelle la composition pharmaceutique est une préparation ophtalmique, de préférence une goutte oculaire.

**10.** Composition pharmaceutique pour une utilisation selon la revendication 9, dans laquelle la goutte oculaire est administrée une fois par jour ou un jour sur deux, avec 1 à 2 gouttes à chaque fois ; de préférence la goutte oculaire est versée dans le sac conjonctival.

**11.** Composition pharmaceutique pour une utilisation selon la revendication 9 ou 10, dans laquelle la goutte oculaire est administrée à un sujet qui est une personne utilisant ses yeux de près ou une personne susceptible d'être myope ;

de préférence la personne utilisant ses yeux de près est un patient myope ou un patient non myope ;
de préférence la personne susceptible d'être myope est une personne âgée de 6 à 18 ans.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150366854 A


### Non-patent literature cited in the description


- Myopia. People's Medical Publishing House, June 2009, 3
- Definition and Classification Criteria for True and False Myopia. 1985
- Myopia. People's Medical Publishing House, June 2009, 25-26
- Blue Book 2015 for Myopia Prevention and Control and Blindness Prevention Model. People's Medical Publishing House, February 2016, 8-9
- Ophthalmology Refractive Optics. Military Medical Science Press, June 2005, 71-72
- Classification of myopia. Ophthalmology and Optometry. People's Military Medical Press, August 2008, 399-401
- Myopia. People's Medical Publishing House, June 2009, 28
- Definition and Classification Criteria for True and False Myopia. Ophthalmology Branch of Chinese Medical Association
- **LIN ZHONG et al.** Research status of transient myopia induced by near-work. *Chinese Journal of Ophthalmology,* July 2012, vol. 48 (7), 657